(19) **Europäisches Patentamt / European Patent Office / Office européen des brevets**

(11) **EP 4 744 672 A1**

(12) # EUROPEAN PATENT APPLICATION

(43) Date of publication:
**20.05.2026 Bulletin 2026/21**

(21) Application number: **24212777.7**

(22) Date of filing: **13.11.2024**

(51) International Patent Classification (IPC):
*A61K 35/28* (2015.01)    *C12N 5/0775* (2010.01)
*A61P 9/10* (2006.01)    *A61P 25/00* (2006.01)
*A61P 25/28* (2006.01)

(52) Cooperative Patent Classification (CPC):
**C12N 5/0663; A61K 35/28; A61P 9/10;
A61P 25/00; A61P 25/28**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **UMC Utrecht Holding B.V.
3584 CS Utrecht (NL)**

(72) Inventors:
• **NIJBOER, Cornelia Henrika Antonia**
**Utrecht (NL)**
• **BENDERS, Maria Johanna Nicole Lilian**
**Utrecht (NL)**
• **VAN BEL, Frank**
**Utrecht (NL)**
• **HEIJNEN, Jacoba Johanna**
**Utrecht (NL)**

(74) Representative: **Algemeen Octrooi- en
Merkenbureau B.V.
P.O. Box 645
5600 AP Eindhoven (NL)**

(54) **INTRANASAL APPLICATION OF MESENCHYMAL STEM CELLS IN THE THERAPY OF NEONATAL HYPOXIC-ISCHEMIC BRAIN INJURY**

(57) The invention relates to mammalian mesenchymal stem cells (MSCs) for use in a method of treatment of a hypoxic-ischemic brain injury (HIBI) event in a human neonate. Particular routes of administration are disclosed. The invention also encompasses the MSCs for use in a method of prevention of the consequences and/or treatment of a neurodevelopmental disability in a neonate which has suffered or is suspected of suffering a HIBI event. A ready to use device for the administering of MSCs to human neonates is also disclosed.

**EP 4 744 672 A1**

**Description**

FIELD OF THE INVENTION

This invention pertains in general to the field of selective therapeutical approaches in particular for the treatment of neonatal hypoxic-ischemic brain injury. It relates to the applications of cell therapy in neonates and to devices for the purpose of the administering of cell therapy in neonates via the intranasal route.

BACKGROUND OF THE INVENTION

The background description includes information that may be useful in understanding the present invention. It is not an admission that any of the information provided herein is prior art or relevant to the presently claimed invention, or that any publication specifically or implicitly referenced is prior art.

Neonatal Hypoxic-ischemic encephalopathy (HIE) is the formal designation according to the ICD10 code (the International Classification of Diseases, Tenth Revision, Clinical Modification) and is a consequence of pre-/perinatal asphyxia (PA) (hypoxia-ischemia) and / or perinatal arterial ischemic stroke (PAIS). Both PAIS and PA are possible causes of hypoxic ischemic brain injury (HIBI), which clinically manifest as a HIE. HIE has one of the most disabling perinatal consequences in term infants, annually affecting 1 to 8 per 1000 children in developed countries and up to 26 per 1000 in underdeveloped countries for PA and 1 per 3000 children in developed countries for PAIS.

Complications related to HIE cause approximately 23% of infant deaths worldwide and affect 0.7-1.2 million newborns annually. Cerebral palsy, epilepsy, intellectual disability, motor and cognitive deficits, learning and behavioral disabilities and other serious neurological deficits are regularly seen in newborns depending on the degree of brain damage.

Infants with mild HIE caused by PA have a mortality of 10%, and 30% suffer from neurodevelopmental disorders/disabilities, while with moderate-severe PA, the mortality is 60% and a majority of the survivors develop severe disability. Hypoxic-ischemic brain injury after perinatal asphyxia lowers the quality of life of children and affects the socioeconomic burden on families, caretakers, and society.

PA (also known as neonatal asphyxia or birth asphyxia) is the medical condition resulting from deprivation of oxygen and/or perfusion to a newborn infant that lasts long enough during the birth process to cause physical harm, usually to the brain. As said, PA can be the cause of a HIBI, or of intraventricular hemorrhage, the latter mainly in preterm births.

Therapeutic hypothermia (TH) became the standard of care to treat newborns with PA after revealing an improved outcome, which reduces death and/or severe disability in the long term. The therapeutic window for TH is 6 h, although TH initiated between 6 h and 24 h after birth may have some beneficial effects. However, TH efficacy has been revealed mostly in babies over 35 weeks of gestational age at birth and when provided in selected centers in developed countries. In addition to those limitations, an unacceptable 40%-50% of PA patients eligible to receive TH still show no benefits from the treatment. Thus, alternative, synergistic or complementary therapies to TH are warranted. Different strategies have been tested in the experimental setting such as erythropoietin, and xenon but they have not demonstrated clinical benefits in humans so far.

PAIS is a cerebrovascular event occurring during fetal or neonatal life, namely within 28 days after birth, with pathological or radiological evidence of focal arterial infarction of brain. PAIS has no treatment. In preclinical studies, melatonin and erythropoietin are effective in reducing brain damage, whereas hypothermia offers just mild and contradictory results. Depending on the extent and location of the stroke, PAIS can lead to lifelong deficits in multiple domains such as motor and cognitive functioning, significantly impacting the quality of life of children and their families. There is a pressing need to develop new therapies for patients with PAIS, as no specific curative treatments are currently available.

Some feasibility and safety studies of autologous cord blood cells in neonates with PA have been conducted with umbilical cord blood cells administered into the bloodstream (see Cotten CM, et al. Feasibility of autologous cord blood cells for infants with hypoxic-ischemic encephalopathy", J Pediatr. 2014 May;164(5):973-979.e1. doi: 10.1016/j.jpeds.2013.11.036. Epub 2013 Dec 31. PMID: 24388332; PMCID: PMC3992180; and Tsuji M, et al. Autologous cord blood cell therapy for neonatal hypoxic-ischaemic encephalopathy: a pilot study for feasibility and safety. Sci Rep. 2020 Mar 12;10(1):4603. doi: 10.1038/s41598-020-61311-9. PMID: 32165664; PMCID: PMC7067794). The authors of these studies concluded that collection, preparation, and infusion of fresh autologous UCB cells for use in infants with PA was feasible. Moreover, no serious adverse events that might be related to cell therapy were recorded.

A feasibility and safety study of autologous cord blood cells in neonates with severe intraventricular haemorrhages was also conducted with umbilical cord blood-derived mesenchymal stem cells (MSCs) administered intraventricularly (see Ahn SY, Chang YS, Sung SI, Park WS. Mesenchymal Stem Cells for Severe Intraventricular Hemorrhage in Preterm Infants: Phase I Dose-Escalation Clinical Trial. Stem Cells Transl Med. 2018 Dec;7(12):847-856. doi: 10.1002/sctm.17-0219. Epub 2018 Aug 21. PMID: 30133179; PMCID: PMC6265626.).

These studies in neonates demonstrate the safety of therapy with mesenchymal stem cells (MSCs). However, the intravenous administering or the administering by means of an intraventricular catheter / transplant are, in any case, complex invasive administration routes, especially in neonates. Additionally in babies with PA with multiorgan damage, the majority of MSCs is lost to other damaged organs instead of receiving the damaged brain, after intravenous administration.

In light of this, new products, compositions, methods and uses for in the treatment of conditions like PA and PAIS, which are causes of HIBI, would be highly desirable but are not yet readily available. In particular, there is a clear need for reliable, efficient, and reproducible products, compositions, methods and uses that allow to be used in the treatment of conditions like PA and PAIS. Accordingly, the technical problem underlying the present invention can be seen in the provision of such products, compositions, methods and uses for complying with any of the aforementioned needs, or at least providing the public with a useful choice. The technical problem is solved by the embodiments characterized in the claims and herein below.

SUMMARY OF THE INVENTION

As embodied and broadly described herein, the present invention is directed to the surprising finding that MSCs, when administered through nasal cavity to human neonates that had suffered from HIBI due to a PAIS or due to PA, do effectively allow a good and better outcome in relation to non-treated neonates.

These results were achieved with a single dose of MSCs intranasally administered (50 million +/- 10 % viable MSCs). Noteworthy is that the MSCs intranasally administered corresponded to doses designed for a previous safety study, which unexpectedly resulted as therapeutically effective. The intranasal administering of the MSCs, besides being a feasible, safe, an non-invasive route of administration, also provided meaningful better neurodevelopmental outcomes. Thus, in the treated human neonates good and improved outcomes of one or more of the following: cerebral palsy, epilepsy, language and/or learning disabilities, visual deficits, and behavioral problems were observed after the treatment according to the invention in relation to non-treated neonates. Moreover, the motor functionality was better preserved in the treated human neonate subjects. In summary, improved long-term (motor)neurodevelopmental outcomes were observed in neonates treated in accordance with the invention, when compared with a group of non-treated controls.

The invention supported in the herewith provided results is noteworthy, since until now, treatment for neonates with PAIS was focused on supportive care, which had no long-term protective or curative effect on the brain.

In view of the prior art, it is noteworthy and a non-expected result that the intranasal administration according to the invention is effective in the treatment of HIBI caused by PA and/or PAIS.

In addition, and as illustrated in the examples, there were no short-term or long-term adverse effects associated to the MSCs (at 4 years) for use as indicated in the present invention.

Intranasal delivery MSCs therapy (in comparison to standard clinical care) has the potential to reduce severe disability following HIBI in neonates.

Thus, in one aspect, the invention relates to mammalian MSCs, preferably human MSCs, for use in a method of treatment of a HIBI event in a mammalian neonate, preferably human neonate, wherein the method comprises the intranasal administration of an effective amount of the MSCs.

This aspect can also be referred to as a method of treating a human neonate subject who has suffered or is suspected of suffering from an HIBI event, the method comprising intranasally administering an effective amount of (human) MSCs, preferably intranasally administering an effective amount of human MSCs in a human neonate in need thereof. In addition, the intranasal administration of an effective amount of the mesenchymal stem cells may be used for HIBI and preventing (the consequences of) conditions of a HIBI, due to for example PA and PAIS.

The invention also encompasses the use of mammalian MCSs, preferably human MSCs, for the preparation of a medicament for the treatment of HIBI in a human neonate, wherein the prevention/treatment comprises the intranasal administration of an effective amount of the MSCs to the human neonate.

With the aim to minimize any inter-species incompatibility, if the mammal to be treated is from a particular specie (e.g. a human) the source of the MSCs to be administered will be from that same specie (e.g. human MSCs).

As will be illustrated in the examples section, the intranasal administration of MSCs, in particular the administration of bone-marrow derived mesenchymal stem cells (BM-MSC) provided improved neurodevelopmental outcome for the neonates in relation to non-treated neonates, at long-term (i.e., two years after the HIBI event). This was achieved even with a single dose of MSCs, which dose was initially selected from studies for the analysis of the safety of the administering of the MSCs. The studies were not conclusive for a therapeutic effect.

As a result of the observed long-term effects after the treatment of the human neonates, it can be concluded that the intranasal administration of MSCs allows to treat and/or prevent neurodevelopmental disabilities caused by the ischemic event (HIBI event), and which disabilities may not be apparent at early months but that can be diagnosed later, for example at 2 years old or even at a later stage. Another aspect of the invention is mammalian MSC, preferably human MSC, for use in a method of treatment of a neurodevelopmental disability in a neonate which has suffered (or is suspected of suffering) a HIBI event, wherein the method comprises the intranasal administration of an effective (i.e. therapeutic) amount of the MSCs.

This aspect can also be formulated as the use of mammalian (preferably human) MSC for the preparation of a medicament for the treatment/prevention of a neurodevelopmental disability in a neonate which has suffered a HIBI event, wherein the method comprises the intranasal administration of an effective (i.e., therapeutic) amount of the MSCs. The invention relates, thus, to a method of treating HIBI and reducing a neurodevelopmental disability in a neonate which has suffered a HIBI event, the method comprising intranasally administering an effective amount of mammalian MSCs, preferably human MSCs if the neonate is a human neonate.

For the intranasal administration of the MSCs, there are tools and devices available that the skilled person will recognize as adequate, and which form part of the known conventional tools or techniques. However, disclosed herein is also a ready to use device that reduces manipulation time and any associated errors in the administering of safe and effective doses/amounts of MSCs.

It is thus another aspect of the invention to provide and use a ready to use device comprising a container and a dispensing element connected to the container, wherein the container comprises a pharmaceutical composition that comprises mammalian MSCs, preferably human MSCs, more preferably human allogeneic MSCs, for single-dose administration of an amount of the cells, preferably for an intranasally single-dose administration of an amount of the cells, preferably in a dose that is effective in a method treatment of an HIBI event in a (human) neonate, as disclosed herein.

DESCRIPTION

*Definitions*

A portion of this disclosure contains material that is subject to copyright protection (such as, but not limited to, diagrams, device photographs, or any other aspects of this submission for which copyright protection is or may be available in any jurisdiction). The copyright owner has no objection to the facsimile reproduction by anyone of the patent document or patent disclosure, as it appears in the Patent Office patent file or records, but otherwise reserves all copyright rights whatsoever.

Various terms relating to the methods, compositions, uses and other aspects of the present invention are used throughout the specification and claims. Such terms are to be given their ordinary meaning in the art to which the invention pertains, unless otherwise indicated. Other specifically defined terms are to be construed in a manner consistent with the definition provided herein. Although any methods and materials similar or equivalent to those described herein can be used in the practice for testing of the present invention, the preferred materials and methods are described herein.

For purposes of the present invention, the following terms are defined below.

As used herein, the singular form terms "a," "an," and "the" include plural referents unless the content clearly dictates otherwise. Thus, for example, reference to "a molecule" includes a combination of two or more molecules, and the like.

As used herein, "about" and "approximately", when referring to a measurable value such as an amount, a temporal duration, and the like, is meant to encompass variations of $\pm 20\%$ or $\pm 10\%$, more preferably $\pm 5\%$, even more preferably $\pm 1\%$, and still more preferably $\pm 0.1\%$ from the specified value, as such variations are appropriate to perform the disclosed invention. Unless otherwise clear from context, all numerical values provided herein include numerical values modified by the term "about."

As used herein, "and/or" refers to a situation wherein one or more of the stated cases may occur, alone or in combination with at least one of the stated cases, up to with all of the stated cases.

As used herein, "at least" a particular value means that particular value or more. For example, "at least 2" is understood to be the same as "2 or more" i.e., 2, 3, 4, 5, 6, 7, 8, 9, 10, 11, 12, 13, 14, 15, ..., etc. Also, for example, "at least 35 weeks"" is understood be the same as "35 or more", i.e., 36, 37, 38, 39, 40, 41, 42, 43, ..., etc.

The term "mesenchymal stem (or stromal) cells" (abbreviated MSCs) relates to multipotent stem cells that can differentiate into various cell types, including bone cells, called osteoblasts, cartilage cells, termed chondrocytes, muscle cells, called myocytes, and fat cells that give rise to adipose tissue, called adipocytes. According to the International Society for Cellular Therapy (ISCT), a cell can be classified as an MSC if it shows plastic adherent properties under normal culture conditions and has a fibroblast-like morphology. Preferably, the cultured MSCs also express on their surface (i.e., membrane) CD73, CD90 and CD105, while lacking the expression of CD11b, CD14, CD19, CD34, CD45, CD79a and HLA-DR surface markers (or cell membrane proteins).

MSCs are found throughout the human body, and a commonly used source is the bone marrow (source of MSCs in the assay disclosed below). The MSCs derived or obtained from the bone marrow of the mammals are also known as bone marrow mesenchymal stromal cells or bone marrow mesenchymal stem cells (abbreviated BM-MSCs). They are also referred in this description as "Bone marrow-derived mesenchymal stem cells". Another source of MSCs is the umbilical cord, and the MSCs derived are known as umbilical cord MSCs (abbreviated (UC-MSCs), and also herewith termed "Umbilical cord-derived mesenchymal stem cells". Indeed, the youngest and most primitive MSCs may be obtained from umbilical cord tissue, namely Wharton's jelly and the umbilical cord blood. Another source of MSCs is the adipose tissue. Adipose-tissue-derived MSCs (abbreviated AdMSCs), in addition to being easier and safer to isolate than BM-MSCs, can be obtained in larger quantities. Although less often used, MSCs can also be derived or obtained from molar cells (i.e., from the developing tooth bud of the mandibular third molar). Finally, the amniotic fluid is also a source of MSCs, wherein 1 in 100 cells collected during amniocentesis are pluripotent mesenchymal stem cells.

When in this description the terms "Bone marrow-derived mesenchymal stem cells" or "Umbilical cord-derived mesenchymal stem cells" or "Adipose-tissue-derived MSCs" are employed, they relate to the MSCs cultured from the bone marrow or from the umbilical cord, or from the adipose tissue respectively, following the well-established methodologies the skilled person will recognize. Independent of the anatomical source, the MSCs applicable according to the present invention are mammalian MSCs, preferably human MSCs. The MSCs isolated or purified from bone marrow, the umbilical cord or any other possible source that are for use according to the invention in the neonates are, preferably, allogeneic cells.

As used herein, the terms "intranasal administration", or "intranasal route" encompass the administering or the delivery of a drug, in particular of a composition that comprises the mammalian (e.g. human) MSCs through the nasal cavity. The intranasal cavity is highly vascularized and may allow efficient transfer of molecules and cells directly to the nervous system via the cribrifrom plate. Compared to other administration routes, nasal drug delivery may increase bioavailability and reduces systemic exposure risks. MSCs intranasally delivered according to this invention includes the administering through a catheter coupled to a syringe. In animal models, the intranasal route enabled MSCs to migrate if HIBI is present directly into the brain and brain-related structures like the meninges, within several hours, thereby preventing the loss of MSCs in other organs (such as the liver and lungs) which is observed after systemic (e.g., intravenous) administration.

As used herein "allogeneic" or "allogenic" (interchangeably used in this description) relate to cells, tissues or any fluid derived from individuals of the same species (e.g., humans) that are sufficiently unlike genetically to interact antigenically. The allogenic MSCs (mammalian/human) are thus cells isolated or contained in any carrier that are derived from an individual, the donor, that is said to be compatible with another individual that will receive such cells, thus the recipient.

As used herein the term "dose" or "amount", when for example it refers to the dose of MSCs, refers to a specific individual amount of these cells, or, in other cases, to any other drug/medication, for example of a pharmaceutical agent, chemotherapeutic agent or ionizing radiation, taken at one time. Typically, a dose is encompassed in a vehicle for administration of the medication (i.e., MSCs optionally in combination with other compounds and carriers) to a subject.

"Neurodevelopmental disorders or disabilities" are associated primarily with the functioning of the neurological system and the nervous system, which includes the brain and spinal cord. According to the American Psychiatric Association Diagnostic and Statistical Manual of Mental Disorders, Fifth Edition, (DSM-5) published in 2013, these conditions generally appear in early childhood, usually before children start school, and can persist into adulthood. All these disorders negatively impact a person's functioning in one or more domains of life (personal, social, academic, occupational) depending on the disorder and deficits it has caused. Examples of neurodevelopmental disorders in children include learning disabilities, intellectual disability (also known as mental retardation), conduct disorders, cerebral palsy and other motor disabilities, attention-deficit/hyperactivity disorder (ADHD), autism, and impairments in vision and hearing. Children with neurodevelopmental disorders can experience difficulties with motor skills, language and speech, behavior, memory, and learning. Epilepsy episodes are also common in children with neurodevelopmental disorders.

As used herein, the term "neurodevelopmental outcome" relates to the manifestation of the brain's ability to learn, focus, develop memories and social skills, and all what a human being, does on a daily basis, which in humans include the tasks as reading, writing, listening, and watching. These abilities result from the development of the brain and neurological system, starting in humans in the fetus phase and continuing after birth and during the whole life.

As used herein, the term "pharmaceutical composition" refers to a composition formulated as pharmaceutically acceptable or physiologically acceptable composition for administration to subject. The compositions of the invention may be administered in combination with other agents as well, provided that the additional agents do not adversely affect the ability of the composition to deliver the intended therapy. The pharmaceutical composition often comprises, in addition to a pharmaceutical active agent, such as in the present case the mammalian MSCs, one or more pharmaceutical acceptable carriers (or excipients). The pharmaceutical compositions can be specially formulated for administration in liquid form, adapted for intranasal administration as, for example, a sterile solution or suspension. The mammalian MSCs of any source in the present invention may be administered suspended in a liquid formulation that comprises the adequate buffers for the preservation of the isotonicity of the cells and assure this way their quality. Other compounds in the pharmaceutical composition include preservative compounds to preserve viability and activity during storage and application All of them will be under the category of "pharmaceutically acceptable" carriers or compounds (see below).

As used herein, the expression "effective amount", when for example referred to the amount of mammalian (e.g., human) MSCs that are for use for the treatment of HIBI in a neonate, include a "therapeutically effective amount" as used herein, but it may also refer to a "preventive effective amount". Indeed, these amounts refer to the amount of a compound that, when administered, is sufficient to prevent development of, or alleviate to some extent, one or more of the symptoms of the disease which is addressed (i.e., HIBI and associated neurodevelopmental disabilities).

When along this description the MSCs are for use in the indicated treatments, it is to be understood that they are useful in the therapy of a manifested condition (i.e., HIBI), but also to prevent this condition in such cases where it is acknowledged as a condition likely to occur. Besides, the terms "treatment" or "treating", as used in this description, also encompass the prevention of severe outcomes due to the manifested condition, as well as the prevention of the development of any neurodevelopmental disability (related to an HIBI event), or the reduction of the extension of this disability.

The term "pharmaceutically acceptable" as used herein pertains to compounds, materials, compositions, and/or dosage forms which are, within the scope of sound medical judgment, suitable for use in contact with the tissues of a subject (e.g. human) without significant toxicity, irritation, allergic response, or other problem or complication, commensurate with a reasonable benefit/risk ratio. Each carrier, excipient, etc., must also be "acceptable" in the sense of being compatible with the other ingredients of the pharmaceutical composition. It must also be suitable for use in contact with the tissue or organ of humans without excessive toxicity, irritation, allergic response, immunogenicity or other problems or complications commensurate with a reasonable benefit/risk ratio. Suitable carriers, excipients, etc. can be found in standard pharmaceutical texts, and include, as a way of example preservatives, agglutinants, humectants, and antioxidants. The skilled person in the art will know the method to determine the said therapeutically effective amount and well as the possible pharmaceutically acceptable carriers or excipients.

As used herein, "conventional techniques" or "methods known to the skilled person" refer to a situation wherein the methods of carrying out the conventional techniques used in methods as disclosed herein will be evident to the skilled worker. The practice of conventional techniques in molecular biology, biochemistry, cell culture, genomics, sequencing, medical treatment, pharmacology, immunology and related fields are well-known to those of skill in the art and are discussed, in various handbooks and literature references.

As used herein, "comprising" or "to comprise" is construed as being inclusive and open ended, and not exclusive. Specifically, the term and variations thereof mean the specified features, steps or components are included. These terms are not to be interpreted to exclude the presence of other features, steps or components. It also encompasses the more limiting "to consist of".

### Detailed description

The invention is defined herein, and in particular in the accompanying claims. Subject-matter which is not encompassed by the scope of the claims does not form part of the present claimed invention.

It is contemplated that any method, use, or composition described herein can be implemented with respect to any other method, use or composition described herein. Embodiments discussed in the context of methods, use and/or compositions of the invention may be employed with respect to any other method, use or composition described herein. Thus, an embodiment pertaining to one method, use or composition may be applied to other methods, uses and compositions of the invention as well.

Any references in the description to methods of treatment refer to the compounds, pharmaceutical compositions, and medicaments of the present invention for use in a method for treatment of the human (or animal) body by therapy.

As embodied and broadly described herein, the present invention is directed to the surprising finding that intranasal administration of human MSCs, is notably effective when administered to human neonates even at a single dose tested for safety purposes. The intranasal administration of the human MSCs decreased brain tissue loss associated to an HIBI event such as a PAIS; and provided improved long-term neurodevelopmental outcomes. No adverse side-effects were observed for the administering of the MSCs by intranasal route.

*Mammalian mesenchymal stem cells (MSC) for use in a method of treatment of a hypoxic-ischemic brain injury (HIBI) event in a human neonate/newborn:*
The invention relates, according to one aspect, to mammalian MSCs for use in a method of treatment of a HIBI event in a mammalian (e.g., human) neonate, wherein the method comprises the intranasal administration of an effective amount of the mesenchymal stem cells.

In an embodiment, the mammalian MSCs are human MSCs, and the neonate is a human neonate.

In another embodiment, the MSCs are, preferably, human-derived mesenchymal stem cells, even more preferably human allogeneic derived mesenchymal stem cells, even preferably human allogeneic bone marrow-derived MSCs.

In another embodiment, the effective amount or dose of MSCs is an amount that prevents (i.e., preventive amount) the consequences of brain injury caused by a possible or already manifested HIBI.

In an embodiment, the effective amount/dose of MSCs is a therapeutic amount/dose that allows the recovery or reduction of the injured brain area, once an HIBI event has been manifested.

For the MSCs for use according to the invention, the age of the neonate is not critical. Effective results have been achieved with neonates of at least 35 weeks of gestation. Thus, in an embodiment, the human neonate is a neonate of at least 35 weeks gestation. In another embodiment is a neonate of at least 35, 36, 37, 38, 39, 40, 41, 42, and 43 weeks gestation.

Tested amounts or doses of MSCs corresponded to amounts designed to check if the proposed route of administration (i.e., intranasal administration) was safe in human neonates. As previously indicated, these amounts showed, moreover, a noteworthy long-term positive outcome for the treated neonates. This makes the invention noteworthy in terms of providing a therapeutic effect with reduced risk of any adverse effect.

Thus, in an embodiment, the amount/dose of MSCs is from about $30 \times 10^6$ to $55 \times 10^6$ cells, preferably from $40 \times 10^6$ to $55 \times 10^6$ cells, preferably from about $45 \times 10^8$ to $55 \times 10^6$ cells. In another embodiment, the amount of MSCs (in a single or in a multiple dose) is selected from about $30 \times 10^6$, $31 \times 10^6$, $32 \times 10^6$, $33 \times 10^6$, $34 \times 10^6$, $35 \times 10^6$, $36 \times 10^6$, $37 \times 10^6$, $38 \times 10^6$, $39 \times 10^6$, $40 \times 10^6$, $41 \times 10^6$, $42 \times 10^6$, $43 \times 10^6$, $44 \times 10^6$, $45 \times 10^6$, $46 \times 10^6$, $47 \times 10^6$, $48 \times 10^6$, $49 \times 10^6$, $50 \times 10^6$, $51 \times 10^6$, $52 \times 10^6$, $53 \times 10^6$, $54 \times 10^6$, $55 \times 10^6$, $56 \times 10^6$, $57 \times 10^6$, $58 \times 10^6$, $59 \times 10^6$ and $60 \times 10^6$ cells. The target dose is preferably of about $50 \times 10^6$ MSCs, with a maximal range of 10% allowed to account for inherent variation in the preparation method.

In yet another embodiment, optionally in combination with any of the embodiments above or below, any of the previously indicated amounts/doses of intranasally administered MSCs is administered to the neonate in a single dose, and preferably by administering nose droplets of a composition comprising the cells over both nostrils and preferably divided equally over the said both nostrils. Preferred volumes for the administering of the amount/dose of MSCs include a volume of at least 0.3 ml, two times, one per each nostril (i.e., 0.6 ml in total). Thus, the total amount of MSCs is, in a preferred embodiment, administered as the sum of MSCs delivered in each of the nostrils. Maximum volumes per nostril are around a value in the range from 0.2 to 0.4 ml per nostril, preferably 0.3 ml per nostril.

In also another embodiment, any of the previously indicated amounts/doses of intranasally administered MSCs is administered to the neonate in multiple doses.

In a more preferred embodiment of the MSCs for use in a method of treatment of a HIBI event in a human neonate according to the invention, the method comprises the intranasal administration of an effective amount MSCs from $30 \times 10^6$ to $60 \times 10^6$ cells, preferably from $45 \times 10^6$ to $55 \times 10^6$ cells; and the MSCs are administered contained in a pharmaceutical composition that is preferably administered in a volume of around 0.3 ml per nostril (0.6 ml in total).

With intranasal administration, it is believed the MSCs migrate from the nasal cavity to the brain and meninges where they can exert their function.

The migration of MSCs may, in the practice of the invention, be allowed by the avoidance of any suction after the MSCs administration.

In an embodiment, the migration is assured by avoiding suction at least from 10 h to 15 h after administration of the MSCs, preferably 12 h after administration of the MSCs.

In another embodiment, optionally in combination with any of the embodiments above or below, the administering is carried out in a mode to avoid swallowing from the neonate. In an embodiment, the composition with the cells is administered dropwise within a time from 10 to 30 seconds.

In yet another embodiment, for the administering of the composition comprising the MSCs to the neonate, the neonate is maintained in midline position, optionally with the head fixed during and after application for a period of time, around 30 to 60 seconds, to check that the composition with MSCs is neither swallowed nor sneezed.

As soon as the HIBI event is diagnosed, an appropriate treatment is proposed with the aim to reduce as much as possible any sequels. Surprisingly, the inventors have found that in particular effective treatment according to the invention can be provided when the intranasal administering of MSCs is within 2-10 days, or within 5 - 10 days, or within 6 - 10 days from the HIBI event, or from the diagnosis of the event. Indeed, as a common procedure during labor, the neonates are monitored and checked to detect any problem. Therefore, in most of the cases the HIBI event is quickly diagnosed and the moment at which it takes place may correspond with the moment of the diagnosis. However, there can be cases in which the HIBI event is neglected due to lack of symptoms and then it is later diagnosed with a few days after birth. In any case, the intranasal administration of the MSCs is at least effective within this first week after the onset of the event. The HIBI event is, in an embodiment, confirmed by neuro-imaging (cranial ultrasound and/or cerebral Magnetic Resonance Imaging - MRI), and then the intranasal administration of MSCs is done, in any case as soon as possible.

It is believed that the sooner the intranasal administration occurs after the HIBI event, the better, since the anti-inflammatory effect next to neuroregeneration of brain injury.

Several scenarios may lead to a HIBI event in neonates. Examples include (a) birth trauma and mechanical trauma to the fetal head and neck that can cause stroke by damaging arteries; (b) trauma to the fetal head from excessive uterine activity, manipulation, pressure, and forceps or vacuum application, perinatal infection, postnatal hypoglycemia, or via direct occlusion or vasospasm that can cause perinatal ischemic stroke (PAIS); (c) birth asphyxia or perinatal asphyxia (PA), and which arises from oxygen deprivation in the womb, and which mainly results from placental abruption, umbilical cord problems, uterine rupture, or the failure to identify abnormal heart rate by the medical staff; (d) congenital heart diseases; (e) intrauterine inflammation that may cause blood clotting or vasospasm occasioning a PAIS; and (f) blood clotting disorders. Often the cause of HIBI is multifactorial.

In a preferred embodiment of the MSCs for use intranasally administered in a neonate that has suffered or is suspected of a HIBI event, the said HIBI event is a HIBI event caused by or coincides with one or more of a PAIS and/or PA.

In another preferred embodiment, the HIBI event is caused by PAIS with ischemic injury to one or more of the corticospinal tracts, cortex, white matter, and central gray matter.

A HIBI event is the damage of a brain area due to the lack of blood and oxygen supply for any reason, in particular those disclosed herein. The result is the loss and death of the brain cells in that blood and oxygen-deprived area. The lack of blood and oxygen supply may be due to the occlusion or vasospasm of the brain arteries. Thus, depending on the brain area where the occlusion and/or the lack of blood/oxygen supply takes place, the HIBI event can also be subclassified.

As previously indicated, there are different possible sources of MSCs. Different sources of MSC are indistinct for use in the method of treatment of the neonates according to the invention. In an embodiment, the source of MSC is selected from the group consisting of bone marrow-derived mesenchymal stem cells (BM-MSC), Umbilical cord-derived mesenchymal stem cells (UC-MSC), and combinations thereof. In an embodiment, the MSCs are BM-MSCs.

As previously indicated the MSCs can be obtained from any possible source by any of the conventional techniques known to the skilled person.

In an embodiment, the selected MSCs express on their surface (i.e., in the cell membrane) the membrane proteins (also known as clusters of differentiation) CD73, CD90, and CD105, and lack the expression on their surface of the membrane proteins CD3 and CD45. Due to the presence or absence of these membrane proteins in the cells, they can also be referred as positive for the membrane proteins CD73, CD90, and CD105, and negative for the membrane proteins CD3 and CD45.

Positive or negative for a surface marker (i.e., in the cell membrane) is to be understood that a marker, such as a protein in the cell membrane, is detected or not detected according to the particular detection limits of a technique used for this aim. The methods to determine the presence (positivity) or absence (negativity) of certain markers in cell types, such as cell membrane proteins that define a particular cell type, form part of the conventional techniques the skilled person will know. An example of technique is the immunophenotyping.

Also provided is for the MSCs for use according to the invention, wherein the MSCs are obtainable or obtained by a method comprising the following steps:

a) Culturing MSCs, preferably human MSCs, from a starting material or source of cells comprising MSCs (which starting material or source may derive from an isolated sample of a subject) in a cell culture expansion medium, preferably in a cell culture expansion medium comprising the amino acid L-glutamine, heparin and a human platelet lysate, wherein the said culturing in an cell culture expansion medium is carried out for a time to reach a first cell confluence state, to obtain a pool of cells that comprise MSCs that are positive for CD73, CD90, and CD105, and that are negative for CD3, and CD45; and

b) Optionally passing the cells of the previous step at least once, preferably two, or three time, preferably in the cell culture expansion medium of the previous step (a), to reach a second or subsequent cell confluence state.

In an embodiment of the MSCs obtained or obtainable by means of the previous method are, in a further step, suspended in a medium for intranasal administration, preferably a liquid (aqueous) medium, and wherein said (liquid) medium for intranasal administration preferably comprises pharmaceutically acceptable salts and proteins. With this further step of suspending the MSCs in a medium for intranasal administration, a pharmaceutical composition for intranasal administration comprising MSCs is obtained.

In an embodiment the (liquid) medium for intranasal administration comprises NaCl at a percentage by weight from 0,7 % w/w to 0.9% w/w, and human serum albumin at a percentage by weight from 15 % w/w to 20% w/w in, relation to the total weight of the liquid medium, and preferably wherein the weight ratio of NaCl: Albumin is preferably of 1:1.

The (liquid) medium for intranasal administration is to be understood as any (liquid) composition that can be intranasally administered according to the regulatory requirements, and that is not prejudicial for the subject. Thus, it is pharmaceutically acceptable.

In an embodiment, the amounts of suspended MSCs in the (liquid) medium for intranasal administration are from $30 \times 10^6$ to $60 \times 10^6$ viable cells, preferably from $40 \times 10^6$ to $55 \times 10^6$ viable cells, preferably from about $45 \times 10^6$ to $55 \times 10^6$ viable cells, in a predetermined total volume aimed for an intranasal administration.

In another embodiment of the MSCs obtained or obtainable as disclosed, the MSCs to be suspended in the (liquid) medium have a viability of at least 70 %, at the moment they are suspended in the liquid medium, said viability measured according to any conventional techniques.

The percentage of viability of the cells, or cell viability, is to be understood as the proportion of a cell type that is alive in a cell culture support (e.g., plate) and can perform its role in physiological conditions. This feature is usually presumed if under the analysis by means of conventional techniques the cells are categorized as alive, or a certain percentage of the cells in a pool or composition are considered alive. Well known examples of cell viability and proliferation assays or techniques for the determining of cell viability include trypan blue cell counting, DNA synthesis proliferation assays, metabolic proliferation assays, and luminescent cell viability assays, among others.

The MSCs for use in a method of treatment of a HIBI event in a human neonate according to the invention are, in an embodiment, administered preferably at utmost 4 hours, after their suspension in the (liquid) medium for intranasal administration as previously disclosed, or which is the same after the composition for intranasal administration comprising MSCs is obtained. Once prepared and suspended in the medium for intranasal administration, the sooner the MSCs are administered the better. Thus, in another embodiment they are administered at utmost 3, 2, 1, and 0.5 hours after being suspended in the (liquid) medium for intranasal administration.

As deduced from the fact that the MSCs to be intranasally administered may be suspended in a (liquid) medium for intranasal administration, it derives that in an embodiment of the MSC for use in a method of treatment of a HIBI in a human neonate, the MSCs are for use intranasally administered as a composition that comprises MSCs, and wherein the composition comprises:

- from 90 % to 100 % of cells that express on their surface the membrane protein CD73, in relation to the total number of cells in the composition;
- from 90 % to 100 % of cells that express on their surface the membrane protein CD90, in relation to the total number of cells in the composition; and
- from 90 % to 100 % of cells that express on their surface the membrane protein CD105, in relation to the total number of cells in the composition.

The pharmaceutical composition for intranasal administration that comprises MSCs for use in the method(s) of the invention, comprises, in an embodiment, residual amounts of other cell types, which may result from the pool of cells from the isolated sample of a mammal, preferably from a human, and that has been used to obtain the MSCs. In an embodiment, the pharmaceutical composition that comprises MSCs comprises from 0.0 % to 0.1 % of cells which are positive for, or that express on their surface, the membrane protein CD3, mainly lymphocytes; and from 0.0 % to 2 % of cells which are positive for, or that express on their surface, the membrane protein CD45, mainly leukocytes.

Herewith disclosed is also a (liquid) composition for intranasal administration that comprises human MSCs, for use in a method of treatment of a HIBI in a human neonate, wherein the composition comprises human MSCs positive for CD73, CD90, and CD105 and, optionally, residual amounts of lymphocytes and other leukocytes.

In yet another embodiment, the composition only comprises MSCs which are characterized by the presence (i.e., expression on their surface), of all the membrane proteins CD73, CD90 and CD105 determined according to conventional techniques.

Herewith disclosed is a composition, preferably a liquid composition that comprises:

(a) MSCs, and
(b) a (liquid) medium for intranasal administration;

wherein the MSCs are positive for, or express on their surface, the membrane proteins CD73, CD90 and CD105, and are negative for, or lack the expression on their surface, of the membrane proteins CD3 and CD45;

wherein the MSCs are in an amount from $30 \times 10^6$ to $60 \times 10^6$ (viable) cells, preferably from $45 \times 10^6$ to $55 \times 10^6$ (viable) cells, in a predetermined total volume of the liquid medium for intranasal administration; and

wherein the medium for intranasal administration is a liquid medium that preferably comprises NaCl at a percentage by weight from 0,7 % w/w to 0.9% w/w, and human serum albumin at a percentage by weight from 15 % w/w to 20% w/w in relation to the total weight of the liquid medium, and preferably wherein the weight ratio of NaCl: Albumin is of 1:1.

The method for preparing the (liquid) composition that comprises MSCs, and a (liquid) medium for intranasal administration comprises suspending the MSCs in said (liquid) medium for intranasal administration following any of the conventional techniques known for the skilled person.

Based on the results obtained by the inventors upon the intranasal administration of MSC, the MSCs are for use in a method of treatment of a HIBI, which is a method of:

- reducing the hypoxic-ischemic injured area after the HIBI event; and/or
- partial or total recovery of tissue in the hypoxic-ischemic injured area after the HIBI event; and/or
- preserving or improving of the cognitive capacity; and/or
- preserving or improving of the motor functionality; and/or
- reducing the incidence or extension of one or more adverse outcome(s) selected from cerebral palsy or other motor dysfunction, epilepsy, language and/or learning disabilities, visual deficits, and behavioral problems.

The term "preserving", as used herein, applied to the cognitive function or to the motor functionality, refers to the maintenance of the functions normally seen in a subject of a particular age without deterioration due to the HIBI.

The term "improving", as used herein, applied to the cognitive function or to the motor functionality, refers to the amelioration of the functions after the HIBI in relation to a previous point in time where these functions were tested, and/or in relation to a non-treated neonate who also suffered a HIBI event at the same time point.

The term "reducing", as used herein, applied to one or more adverse outcome(s), refers to the lowering of the incidence of these adverse effects in relation to a non-treated neonate who also suffered a HIBI. It also refers to the extension or grade of these adverse effects in case they are given in relation to a non-treated neonate.

The cognitive capacity or cognitive skill of a human subject relates to the skills of the mind, as opposed to other types of skills such as motor skills. The cognitive capacity may include literacy, self-reflection, logical reasoning, abstract thinking, critical thinking, introspection, memory, executive functions and mental arithmetic. Cognitive skills vary in processing complexity and can range from more fundamental processes such as perception and various memory functions, to more sophisticated processes such as decision making, problem solving and metacognition. There are several tests and methods to determine the cognitive capacity of a subject. Obviously, in the case of children the tests are adapted to their age.

In an embodiment, the cognitive functionality is determined by the analysis of one or more of language delay, and cognitive delay (all these delays in relation to the admitted as normal at the age of the children).

The motor functionality involves specific movements of the body's muscles to perform a certain task. It includes gross motor skills and fine motor skills. Gross motor skills require the use of large muscle groups in the legs, torso, and arms to perform tasks such as: walking, balancing, and crawling. Fine motor skills require the use of smaller muscle groups to perform smaller movements. These muscles include those found in our wrists, hands, fingers, feet and in our toes.

In an embodiment, the motor functionality is determined by the analysis of one or more of motor delay in relation to the subjects of the same age, and/or age to start walking.

The cognitive capacity and/or the motor functionality are, in an embodiment, evaluated using standard methodologies known in the art and field. In an embodiment these standards are selected from one or more of the Gross Motor Function Classification System (GMFCS) for classification of cerebral palsy, the Bayley scales for Infant and Toddler Development - version 3 (BSID-III-NL, >2009, see van Baar AL, Steenis LJP, Verhoeven M. Bayley-III-NL; technische handleiding. Pearson assessment and information. B.V., Amsterdam, the Netherlands, 2014.), the Griffiths Scales of Child Development 3rd Edition (see Stroud, L., Foxcroft, C., Green, E., Bloomfield, S., Cronje, J., Hurter, K. et al. (2016). Griffiths Scales of Child Development 3rd Edition. Hogrefe), Child Behavior Checklist (CBCL), Wechsler Preschool and Primary Scale of Intelligence - Fourth Edition (WPPSI-IV) and Wechsler Intelligence Scale for Children - Fifth Edition (WISC-V) standardized lexical development questionnaires, the hammersmith infant neurological exam, and Prechtl's general movements assessment including Motor Optimality Score Revised (MOS-R) and Movement Assessment battery for Children (M-ABC).

In another embodiment, several parameters of the cognitive capacity and/or the motor functionality are assessed, and they are converted to Z-scores to be able to compare different assessment methods or standards. The skilled person understands how to do so.

Cerebral palsy is a group of movement disorders that appear in early childhood, and which signs and symptoms vary among people and over time. They include mostly poor coordination, stiff muscles, weak muscles, and tremors. There may be problems with sensation, vision, hearing, and speech. Babies with cerebral palsy may have difficulties to crawl or walk.

Epilepsy is a term that encompasses a group of non-communicable neurological disorders characterized by recurrent epileptic seizures. An epileptic seizure is the clinical manifestation of an abnormal, excessive, and synchronized electrical discharge in the neurons. Epileptic seizures can result in physical injuries. In epilepsy, seizures tend to recur and may have no detectable underlying cause.

Language and/or learning disabilities encompasses those disorders associated to language development, learning development and language associated learning developments. Examples include specific language impairment, better defined as developmental language disorder, or DLD, aphasia, dyslexia, dysgraphia, auditory processing disorder (APD), language processing disorder, and nonverbal learning disabilities (NVLD), among others.

Visual deficits include a broad spectrum of visual impairment outcomes, in which visual perception is partial or totally lost. For example, a visual deficit is a visual field defect, in which the person's scope of vision while the eyes are focused on a central point is not complete because the nerves that carry visual signals from the back of the eye to the brain's visual processing center are damaged, and part of the visual field disappears.

Behavioral problems or disorders refer to any conduct in children that sometimes argue, are aggressive, or act angry or defiant around adults in an uncommon way for the child's age at the time, persist over time, or are severe. Examples include the oppositional defiant disorder (ODD), conduct disorder (CD) attention deficit hyperactivity disorder (ADHD), Anxiety, Autism spectrum disorder, and sensory processing disorder.

As previously indicated for the cognitive capacity and motor functionality, the language and/or learning disabilities, the visual defects and/or the behavioral problems/disorders are, in a particular embodiment, analyzed following standard methods which optionally allow for scoring at several time points within the analysis.

The MSCs for use according to the aspect of the invention, for the intranasal administration in a human neonate in case of a HIBI event or suspect thereof, is also contemplated in combination with other preventive and/or therapeutic approaches once the HIBI takes place or is diagnosed.

Thus, in another embodiment, the treatment further comprises the administration of a further pharmaceutical drug or agent, preferably wherein the agent is selected from the group consisting of erythropoietin, brain-derived neurotrophic factor, vascular endothelial growth factor, insulin-like growth factor, glial-derived neurotrophic factor, melatonin, allopurinol, xenon, and combinations thereof.

As mentioned, one of the most extended therapeutic approaches in case of a HIBI event caused by perinatal asphyxia in neonates includes therapeutic hypothermia, according to which the body temperature is maintained low for a specific duration of time in an effort to improve health outcomes during recovery after a period of stopped blood flow and oxygen to the brain. The target temperature is often between 32 and 34 °C, such as 33.5 °C in infants and newborns. This treatment has been shown to reduce mortality and cerebral palsy and neurological deficits in survivors of a HIBI event caused by perinatal asphyxia.

The MSCs for use according to the invention, comprises in an embodiment, the treatment performed in combination with therapeutic hypothermia. In a preferred embodiment, the therapeutic hypothermia is performed prior to the administration of the intranasal MSCs. In another embodiment, the therapeutic hypothermia is performed simultaneously.

When therapeutic hypothermia is carried out in combination, the therapeutic window comprises the start of therapeutic hypothermia within at least 6 hours after birth with a HIBI event caused by PA, preferably at a temperature between 32 and 34 °C , such as 33.5 °C; maintenance of therapeutic hypothermia for a period of time from 70 -75 hours, preferably 72 hours; rewarming of the neonate/newborn to physiological temperature (35 - 36 °C) for a period of time of at least 12 hours; and intranasally administering of the MSCs after the rewarming, preferably 5 days after the HIBI event.

*Mammalian mesenchymal stem cells (MSC) for use in a method of prevention and/or treatment of a neurodevelopmental disability:*

As a result of the observed long-term effects after the treatment of the human neonates according to the invention, it is concluded by the inventors that the intranasal administration of MSCs allows treating or reducing the impact/outcome of neurodevelopmental disabilities caused by the hypoxic-ischemic event (for example HIBI event), which disabilities may not be apparent at early months but that can be diagnosed later.

Therefore, another aspect of the invention relates to mammalian/human mesenchymal stem cells (MSC) for use in a method of treatment of a neurodevelopmental disability in a neonate which has suffered or is suspected of suffering a HIBI event, wherein the method comprises the intranasal administration of an effective amount of the MSCs.

The effective amount is a therapeutic amount that prevents the development of any neurodevelopmental disability (related to the HIBI event), or that reduces the extension of this disability, and preferably includes those effective amounts of MSCs disclosed herein.

Examples of neurodevelopmental disabilities according to the invention include those selected from one or more of cerebral palsy or other motor dysfunction, cognitive dysfunction, epilepsy, visual field deficits, language and/or learning disabilities, and behavioral problems. All of them have been disclosed before in this description, as well as the common and conventional modes to analyze them.

The embodiments listed and commented in the context of the previous aspect of the invention, relating to the MSCs for use in a method of treatment of a HIBI event in a human neonate, do also apply to this aspect of the invention, i.e. to mammalian/human MSCs for use in a method of treatment of a neurodevelopmental disability in a neonate which has suffered a HIBI event.

Thus, for this other aspect of the invention, MSCs are selected from the group consisting of BM-MSC, UC-MSC, and combinations thereof. In a preferred embodiment, the MSCs are BM-MSCs.

In another embodiment of the MSCs for use in a method of treatment of a neurodevelopmental disability according to the invention, optionally in combination with any of the embodiments of this aspect, the human neonate is a neonate of at least 35 weeks gestation; and/or the neonate is administered a (single) dose of MSCs from $30 \times 10^6$ to $60 \times 10^6$ cells, preferably from $40 \times 10^6$ to $55 \times 10^6$ cells, preferably from about $45 \times 10^6$ to $55 \times 10^6$ cells; and/or the intranasal administration is performed within 2-10 days from (diagnosis of) the HIBI event, and/or the HIBI event is caused by one or more of a perinatal artery ischemic stroke (PAIS), and a perinatal asphyxia (PA).

*Ready to use device and use thereof*

Herewith disclosed is, as previously indicated, a ready to use device comprising a container and a dispensing element connected to the container, wherein the container comprises a pharmaceutical composition that comprises mammalian MSCs, preferably human MSCs, more preferably human allogeneic MSCs for a single-dose administration of an amount of the cells, preferably for an intranasally single-dose administration of an amount of the cells, preferably in accordance with the methods of treatments disclosed herein.

In an embodiment of the ready to use device, the MSCs are selected from UC-MSCs or BM-MSCs, and they are preferably allogenic BM-MSCs.

The ready to use device according to the invention comprises, in an embodiment, a container that is preferably a syringe with a volume from 0.6 to 2.0 ml, preferably with a volume of 1.0 ml, and the dispensing element is a catheter of a length of utmost 2 cm connected to the syringe.

In yet another embodiment, the container comprises from 0.3 to 2.0 ml, preferably from 0.3 to 1.0 ml, and more preferably from 0.3 to 0.6 ml of the pharmaceutical composition that comprises mammalian MSCs, preferably human MSCs, more preferably human allogeneic MSCs, preferably MSCs selected from UC-MSCs or BM-MSCs.

The device is, in an embodiment, designed for the administration of the adequate dose to each of the nostrils, in such a way that the device comprises at least the total of the volume of the pharmaceutical composition to be administered to the two nostrils. In the alternative, the device is designed to comprise only the volume of the composition for one of the nostrils, meaning that two devices are required for the administering of the MSCs in both of the nostrils. In any of the embodiments, the aim of the device is to assure the administration of the adequate dose of the cells to the subject (i.e., neonate). Therefore, in some examples, the effective dose of MSCs is comprised in the device according to the invention in a volume that is intended to be administered intranasal. For example, in some examples, the device according to the invention may comprise a volume of 1 ml of the pharmaceutical composition, and wherein 0.6 ml comprises an amount of cells that is considered an effective amount of MSCs in the context of the current invention.

Finally, also contemplated is a ready to use device as defined in the previous, for use in a method of treatment of a hypoxic-ischemic brain injury (HIBI) event in a human neonate, and/or for use in a method of treatment of a neurodevelopmental disability in a human neonate, and as described herein in detail.

The foregoing description of the specific embodiments will so fully reveal the general nature of the invention that others can, by applying knowledge within the skill of the art (including the contents of the references cited herein), readily modify and/or adapt for various applications such specific embodiments, without undue experimentation, without departing from the general concept of the present invention. Therefore, such adaptations and modifications are intended to be within the meaning and range of equivalents of the disclosed embodiments, based on the teaching and guidance presented herein.

All references cited herein, including journal articles or abstracts, published or corresponding patent applications, patents, or any other references, are entirely incorporated by reference herein, including all data, tables, figures, and text presented in the cited references. Additionally, the entire contents of the references cited within the references cited herein are also entirely incorporated by references.

It is to be understood that the phraseology or terminology herein is for the purpose of description and not of limitation, such that the terminology or phraseology of the present specification is to be interpreted by the skilled artisan in light of the teachings and guidance presented herein, in combination with the knowledge of one of ordinary skill in the art.

It will be understood that all details, embodiments, and preferences discussed with respect to one aspect of embodiment of the invention is likewise applicable to any other aspect or embodiment of the invention and that there is therefore not need to detail all such details, embodiments, and preferences for all aspect separately.

Having now generally described the invention, the same will be more readily understood through reference to the following examples which are provided by way of illustration and are not intended to be limiting of the present invention. Further aspects and embodiments will be apparent to those skilled in the art.

EXAMPLES

***Example***

Perinatal Arterial Stroke treated with intranasally administered MSCs is safe and provides improved neurodevelopmental outcomes at two years of age in relation to non-treated controls.

The following example illustrates several aspects of the invention. In it the methods and materials to administer allogeneic human BM-MSCs, as well as the technologies employed to determine the extent of a HIBI in neonates are disclosed. Furthermore, the relevant results obtained are discussed.

In an earlier study, the inventors demonstrated that nasal administration MSCs (IN-MSCs) within one week after birth was feasible and safe up to three months of age in ten infants with PAIS.(Baak LM, Wagenaar N, van der Aa NE, Groenendaal F, Dudink J, Tataranno ML, et al. Feasibility and safety of intranasally administered mesenchymal stromal cells after perinatal arterial ischaemic stroke in the Netherlands (PASSIoN): a first-in-human, open-label intervention study. Lancet Neurol. 2022;21 (6):528-36)). The results of this study are not conclusive on therapy and efficacy.

With the assessment of the long-term safety of MSC treatment and the analysis of the neurodevelopmental outcomes of the ten study participants at 2 years of age, the inventors have now noteworthy found that doses for the safety assessment are safe on the long-term and provide evidence of improvements after a HIBI event, for example caused by PAIS. For this, the MRI and neurodevelopmental outcome characteristics of the study participants were compared with a registry cohort consisting of non-treated children from the Neonatal Stroke Registry Utrecht, who would have been eligible for study participation based on the inclusion and exclusion criteria of the PASSIoN trial but were born outside the study period.

As an additional measure of safety, the inventors present a quantitative analysis of brain tissue loss after PAIS of these participants using MRI scans in the first week of life, and at 3 months of age.

Methods

Study Participants:

In the PASSIoN trial, ten neonates were prospectively enrolled in an open-label nationwide phase I intervention study, after written parental consent (Baak et al, *supra*). This trial was conducted in the Netherlands from February 2020 until July 2021, and was approved by the Dutch Central Committee on Research Involving Human Subjects (CCMO, NL59265.000.16). All participants were born ≥36 weeks of gestation and had MRI-confirmed PAIS in the middle cerebral artery (MCA) region with involvement of the cortex, white matter, corticospinal tracts, and basal ganglia. The infants received $50 \times 10^6$ bone marrow-derived MSCs intranasally at the neonatal intensive care unit (NICU) of the Wilhelmina Children's Hospital (WCH) in Utrecht, the Netherlands, within 7 days of presenting signs of PAIS.

The inventors compared the MRI and neurodevelopmental outcomes with a cohort from the Neonatal Stroke Registry Utrecht (NSRU), a database that includes clinical, neuroimaging and neurodevelopmental outcome data of all children admitted to the WCH with PAIS born from 1990 onwards. The inventors selected a subset of the NSRU participants who would have been eligible to participate in the trial based on the inclusion and exclusion criteria, if the trial had been enrolling participants at the time of their birth. These criteria included a similar extent of the MCA stroke based on neonatal MRI, involving the white matter, deep and cortical gray matter, and corticospinal tracts. A part of this registry cohort received 3000 IU/kg erythropoietin intravenously in the neonatal period as part of their participation in a phase I safety and feasibility intervention trial (see Benders MJ, et al. Feasibility and safety of erythropoietin for neuroprotection after perinatal arterial ischemic stroke. J Pediatr [Internet]. 2014 Mar [cited 2020 Feb 4];164(3):481-2. Available from: http://www.ncbi.nlm.nih.gov/pubmed/24321539). Both the parents and/or patients (depending on their age) of the registry cohort gave written consent to collect their neonatal and follow-up data in the NSRU and to use these data for future research.

Next, Table 1 illustrates the patient characteristics, MRI, and neurodevelopmental outcome characteristics of the 10 infants of the test (PASSIoN) that received mesenchymal stromal cells intranasally, when born and at the follow-up.

Table 1. (Values are depicted as n (%), mean±SD or median (IQR). ASM= anti-seizure medication, HINE = Hammersmith Infant Neurological Examination. * n=8, missing data of two children)

| | PASSIoN (n=10) |
|---|---|
| **Neonatal characteristics** | |
| Sex | |
| Male | 4 (40) |
| Female | 6 (60) |
| Gestational age, weeks | 40.29 (1.43) |
| Birthweight, grams | 3492±460 |
| Apgar score at 1 min | 6 (5) |
| Apgar score at 5 min | 8 (2) |
| Seizures | 9 (90) |
| Age at onset of symptoms < 48h | 10 (10) |
| Perinatal asphyxia | 1 (10) |
| Number of ASM received | 3 (2) |
| Day of MSC treatment, days | 6.1 (1.2) |
| **Neurodevelopmental follow-up** | |
| Age at last follow-up (months) | 24.3 (0.9) |
| CP, n (%) | 2 (20) |
| Motor delay, n (%) | 0 (0) |
| HINE Total score* | 78 (1) |

(continued)

| Neurodevelopmental follow-up | |
|---|---|
| HINE number of asymmetries* | 0.5 (2) |
| Age when started walking (months) | 14 (3) |
| Ipsilesional hand preference, n (%) | 6 (60) |
| Bayley-III fine motor Z-score | 0.83 (1.08) |
| Bayley-III gross motor Z-score | -0.33 (1) |
| Bayley-III motor composite score | 104 (13) |
| Bayley-III cognitive composite score | 106 (3) |
| Cognitive delay, n (%) | 1 (10) |
| Language delay, n (%) | 2 (20) |
| Epilepsy, n (%) | 0 (0) |
| Behavioral problems, n(%) | 1 (10) |
| Visual field defect, n (%) | 0 (0) |
| Number of domains with adverse outcome, n (%) | 1 (2) |
| Children with $\geq$ 1 adverse outcome, n (%) | 5 (50) |

Table 2 shows in more detail the patient characteristics of the patients of the PASSIoN trial and the control (registry) cohorts.

Table 2: Patient characteristics of the infants from the PASSIoN cohort and the database cohort (n=49) (Values are depicted as n (%), mean±SD or median (IQR). ASM= anti-seizure medication, HINE = Hammersmith Infant Neurological Examination. * n=8, missing data of two children)

| | PASSIoN (n=10) | Registry (n=39) | p-value* |
|---|---|---|---|
| Sex | | | |
| Male | 4 (40) | 23 (59) | |
| Female | 6 (60) | 16 (41) | .311 |
| Gestational age, weeks | 40.29 (1.43) | 39.86 (2.43) | .464 |
| Birthweight, grams | 3492±460 | 3325±415 | .273 |
| Delivery mode | | | |
| Vaginal | 7 (70) | 21 (54) | |
| Elective C-section | 0 (0) | 2 (5) | |
| Emergency C-section | 3 (30) | 16 (41) | .570 |
| Apgar score at 1 min** | 6 (5) | 6 (5) | .979 |
| Apgar score at 5 min *** | 8 (2) | 8 (3) | .907 |
| Seizures | 9 (90) | 38 (97) | .370 |
| Age at onset of symptoms < 48h | 10 (10) | 30 (77) | .243 |
| Hypoglycemia**** | 1 (90) | 9 (23) | .420 |
| Perinatal asphyxia | 1 (10) | 6 (15) | 1.0 |
| Therapeutic hypothermia | 0 (0) | 2 (5) | 1.0 |
| Number of ASM received | 3 (2) | 3 (2) | .211 |

MRI characteristics:

Infants from the current study cohort and the Registry cohort underwent a neonatal MRI, performed within 10 days after the onset of symptoms, and a follow-up MRI around 3 months of age as part of standard clinical care. All infants were scanned on a 1.5 or 3T Tesla Philips whole-body Achieva system including 8-channel head coil (Philips Medical Systems, Best, Netherlands), with at least a 2 mm axial T1WI, a 2 mm axial T2WI, and a 3 mm diffusion-weighted sequence (scanning characteristics were reported previously by Kersbergen KJ, et al. Microstructural brain development between 30 and 40 weeks corrected age in a longitudinal cohort of extremely preterm infants. Neuroimage 2014;103:214-24. Available from: http://dx.doi.org/10.1016/j.neuroimage.2014.09.039). During the MRI, infants wore hearing protection, and were wrapped in a vacuum pillow to avoid movement during the procedure. The infants who were not sleeping received choral hydrate orally as a mild sedative under respiratory monitoring.

MRI analysis:

The affected arterial stroke territory and the involvement of several brain areas including those involved in motor function (posterior limb of the internal capsule; PLIC, cerebral peduncle) were qualitatively scored on both MRI scans (neonatal and follow-up) by neonatologists with expertise in neonatal neurology (JD, MJNLB) following a previously described method (see Baak et al, supra). Additionally, we assessed whether the perirolandic area, a region where the primary motor and sensory cortex are located, was part of the primary stroke lesion.

As an additional safety precaution, several stopping rules were predefined in the study protocol. The last stopping rule stated that the study should be stopped if the region of infarction increased by >20% after treatment with MSCs in three different patients, expressed as a tissue loss ratio of >120%. Therefore, we assessed the TLR following PAIS by comparing the patient's brain volumes from neonatal and follow-up MRI scans. The calculations performed to produce the TLR can be found at the end of this section. For quantitative analysis of the stroke lesion, we used an in-house developed segmentation method that measured brain tissue and stroke volumes (see Zoetmulder R, et al. Brain segmentation in patients with perinatal arterial ischemic stroke. Neuroimage Clin. 2023 Jan 1;38.). This method uses convolutional neural networks (CNNs) to segment the stroke lesion and 14 major tissue classes based on the neonatal DWI and T2 sequence. Segmentation quality was visually assessed, and if necessary, manually adjusted using ITK- SNAP (version 3.8.0) (Yushkevich PA, et al. User-guided 3D active contour segmentation of anatomical structures: Significantly improved efficiency and reliability. Neuroimage. 2006 Jul 1;31(3):1116-28.). The inventors expressed the stroke volume relative to the total brain volume (TBV), which consists of the total intracranial volume without cerebrospinal fluid and ventricle volumes. The inventors were not able to assess the tissue loss ratio of the patients of the registry cohort due to the large variety in scan quality and scan protocols.

Although the summarized results for the test cohort have already been illustrated in Table 1, next Table 3 illustrates the MRI characteristics of the infants of the test and control (registry) cohorts.

Table 3. MRI characteristics of the infants from the current trial, and a database cohort adhering to the trial inclusion criteria (n=49) (Values are depicted as n(%), or median (IQR). MCA = middle cerebral artery, CST = corticospinal tract, PLIC = posterior limb of the internal capsule. * p-values were calculated by a Mann Whitney-U, a Chi square, or a Fisher's exact test, ** n=36 in the database cohort)

| | | PASSIoN (n=10) | Registry (n=39) | p-value* |
|---|---|---|---|---|
| **Neonatal MRI** | | | | |
| Age at MRI, days | | 4.5 (3) | 4 (3) | .84 |
| Stroke side | | | | .19 |
| | Left | 7 (70) | 19 (49) | |
| | Right | 2 (20) | 19 (49) | |
| | Bilateral stroke | 1 (10) | 1 (3) | |
| Stroke territory | | | | .06 |
| | Anterior branch MCA | 1 (10) | 4 (10) | |
| | Main MCA | 1 (10) | 18 (46) | |
| | Middle branch MCA | 4 (40) | 6 (15) | |
| | Posterior branch MCA | 3 (30) | 11 (28) | |
| | Cortical stroke in MCA | | | |
| territory | | 1 (10) | 0 (0) | |
| Perirolandic area | | 7 (70) | 35 (90) | .14 |
| CST involvement | | 10 (100) | 39 (100) | 1.0 |
| | PLIC | 10 (100) | 38 (97) | 1.0 |
| | Cerebral peduncle | 8 (80) | 34 (87) | .62 |
| Basal ganglia | | 5 (50) | 25 (64) | .48 |
| Thalamus | | 9 (90) | 34 (87) | 1 |
| Basal ganglia and thalamus | | 4 (40) | 22 (56) | .48 |
| Multiple ischemic lesions | | 8 (80) | 20 (51) | .16 |
| **Follow-up MRI** | | | | |

(continued)

| Multiple ischemic lesions | 8 (80) | 20 (51) | .16 |
|---|---|---|---|
| Age at MRI, days | 87 (11) | 96 (14) | .13 |
| Perirolandic area** | 5 (50) | 33 (92) | .007 |
| CST asymmetry** | 5 (50) | 30 (83) | .04 |
| PLIC** | 4 (40) | 29 (81) | .02 |
| Cerebral peduncle** | 1 (10) | 22 (61) | .01 |
| Basal ganglia** | 4 (40) | 16 (44) | 1.0 |
| Thalamus** | 3 (30) | 20 (56) | .28 |
| Basal ganglia+thalamus** | 2 (20) | 15 (42) | .28 |

Neurodevelopmental outcome assessments:

All patients with HIBI receive routine clinical follow-up visits at 4, 9-12, and 24 months of age at the neonatology outpatient clinics of the WCH or one of the other Dutch NICUs. During the visit at two years of age, the patient was assessed by a neonatologist, a physiotherapist, and a pediatric psychologist, providing outcome information on CP, motor and cognitive performance, and language developmental delays, behavioral problems, epilepsy, and visual field defects. To evaluate these outcomes, we performed a neurodevelopmental examination including the Gross Motor Function Classification System (GMFCS) classification when CP was present, and either the Bayley scales for Infant and Toddler Development - version 3 (BSID-III, >2009) or the Griffiths Scales of Child Development 3rd Edition (all patients in the NSRU born before 2009). Language developmental delays were assessed using a lexical development questionnaire or by assessment of the neonatal follow-up team. Behavioral problems were identified with the Child Behavior Checklist (CBCL), and visual field defects were examined by an ophthalmologist if optic radiation injured was detected on follow-up MRI. The occurrence of any serious adverse events was monitored by asking parents about symptoms, diagnoses, hospital admissions, and treatments by other healthcare professionals, and by performing a neurological examination. All continuous outcome parameters were converted to Z-scores to be able to compare different assessment methods. A Z-score below -1 was considered a developmental delay.

Next Table 4 illustrates in detail the neurodevelopmental outcomes observed at two years of age of the infants.

Table 4. Neurodevelopmental outcomes at two years of age of the infants from the current PASSIoN trial cohort, and the database registry cohort (n=49). (Values are depicted as n(%), or median (IQR). CP = cerebral palsy, GMFCS = gross motor function classification system. * p-values were calculated by a Mann Whitney-U or a Fisher's exact test, ** n=38 in the database cohort)

| | | PASSIoN (n=10) | Registry (n=39) | p-value* |
|---|---|---|---|---|
| Age at last follow-up (months) | | 24.3 (0.9) | 23.7 (4.9) | .20 |
| CP, n (%) | | 2 (20) | 19 (49) | .16 |
| | GMFCS I** | 2 (20) | 12 (32) | 1.0 |
| Motor performance (Z-score) | | 0.27 (0.83) | -0.42 (1.50) | **.003** |
| Motor delay, n (%) | | 0 (0) | 13 (33) | **.045** |
| Age when started walking (months) | | 14.3 (3.0) | 17.0 (8) | **.006** |
| Ipsilesional hand preference, n (%) | | 6 (60) | 29 (74) | .44 |
| Cognitive performance (Z-score) | | 0.37 (1.67) | -0.42 (1.19) | .12 |
| Cognitive delay, n (%) | | 1 (10) | 8 (21) | .66 |
| Language delay, n (%) | | 2 (20) | 10 (26) | 1.0 |
| Epilepsy, n (%) | | 0 (0) | 5 (13) | .57 |
| Behavioral problems, n (%) | | 1 (10) | 4 (10) | 1.0 |
| Visual field defect, n (%) | | 0 (0) | 14 (41) | **.02** |
| | Hemianopia | | 8 (21) | |
| Number of domains with adverse outcome, n (%) | | 1 (2) | 0.5 (1) | .11 |
| Children with ≥ 1 adverse outcome, n (%) | | 5 (50) | 26 (67) | .47 |

Statistical analysis:

We tested the data for normality by visual inspection, Levene's test for normality, and the Shapiro-Wilk test. Because of the small sample size, we handled missing data by pairwise deletion. Continuous parameters were depicted as mean and standard deviation (SD), or median and interquartile ranges (IOR), depending on normality of the data. Categorical parameters were depicted as numbers and percentages. We compared clinical and MRI characteristics of the infants from the PASSIoN cohort with the registry cohort by using either independent samples t-test, Mann Whitney-U, Fisher's Exact test, or a Chi-Square test, where applicable.

Tissue loss ratio analysis:

As previously indicated, the inventors developed a quantitative analysis of brain tissue loss after PAIS using MRI scans in the first week of life, and at 3 months of age.

The inventors calculated the tissue volume of both cerebral hemispheres at both neonatal and follow-up timepoints, and the stroke volume of the neonatal MRI. The total brain volumes did not include the volume of cerebrospinal fluid outside the cerebral cortex, inside the ventricles and/or cysts, and volumes of the brainstem, corpus callosum, and cerebellum. In case of a large stroke, the acute swelling on the neonatal MRI can result in an asymmetry of the hemispheres. The inventors previously noticed asymmetries up to 30% (unpublished data), whereas this degree of asymmetry is not observed in a healthy children. In the calculation, we therefore made the assumption that the neonatal affected and unaffected hemisphere had an equal volume.

First, the growth ratio (GR) of the brain between both timepoints was calculated, by comparing the volume of the unaffected, contralesional hemisphere on the neonatal MRI ($V_{C1}$) and the follow-up MRI ($V_{C2}$).

$$GR = V_{C2} / V_{C1}$$

Next, the growth ratio was used to calculate the expected volume of the stroke ($V_{S2\_exp}$) on the follow-up MRI based on the neonatal stroke volume. It was assumed that the tissue affected by the stroke ($V_{S1}$) on the neonatal scan would have dissolved completely.

$$V_{S2\_exp} = V_{S1} * GR$$

To measure the amount of volume lost at the follow-up MRI, we subtracted the contralesional hemispheric volume ($V_{C2}$) from the affected ipsilesional hemispheric volume ($V_{I2}$). Assuming that both hemispheres would have had the same volume on the neonatal scan and the growth rate would have been similar, $V_{S2\_meas}$ represents the tissue which was lost due to the stroke, or 'measured stroke'.

$$V_{S2\_meas} = V_{C2} - V_{I2}$$

Finally, a ratio between the 'measured stroke' and the 'expected stroke', the tissue loss ratio (TLR), was determined.

$$TLR = V_{S2\_meas} / V_{S2\_exp} \times 100$$

A TLR of 100% indicates that a similar amount of cerebral tissue was lost as a result of the stroke as was to be expected based on the neonatal stroke volume, a ratio above 100% suggests that more tissue is lost than expected, and a ratio below 100% suggests that more tissue was preserved. Since we used contralesional hemispheric growth as an internal control for each child, we did not adjust the hemispheric volumes for postmenstrual age at either MRI time point.

The volumes of the stroke and both hemispheres were extracted from these two MRI scans. The volumes of the contralesional hemisphere on the neonatal ($V_{C1}$) and the follow-up MRI ($V_{C2}$) were used to calculate the brain's growth ratio. The growth ratio was used to calculate the expected volume of the stroke on the follow-up MRI, based on the initial stroke volume ($V_{S1}$). The amount of lost tissue on follow-up MRI was calculated by subtracting the volume of the ipsilesional hemisphere at three months of age ($V_{I2}$) from $V_{C2}$.

Production and characterization of MSCs:

For MSC treatment in the 10 neonates in the PASSIoN trial, MSCs derived from allogeneic bone marrow from a healthy 12- year-old male donor were used (approved by the CCMO; NL41015·041·12). MSCs were expanded in CellSTACK® culture chambers (Corning, ME, USA) with Minimum Essential Medium α (MEMα) medium with L-glutamine (Macopharma, Mouvaux, France) supplemented with heparin (LeoPharma, Breda, The Netherlands) and human platelet lysate (MultiPL'100i; Macopharma). MSCs were passaged upon reaching near confluence using TrypLE Select (GIBCO, Life Technologies, NY, USA), and cryopreserved after three passages at a dose of $1 \times 10^8$ cells in NaCl containing 10% human serum albumin and 10% dimethyl sulfoxide. Immunophenotyping was performed by staining cells with CD3, CD45, CD73, CD90, and CD105 conjugated antibodies1 (Beckton Dickinson (BD), Franklin Lakes, NJ, USA) for 20 minutes at 4 °C in the dark. Dead cells were excluded using 7-Amino-Actinomycin D. Samples were washed and analyzed using a FACSCanto flow cytometer (BD) with the BD FACSDiva Software.

MSC production was performed aseptically under conditions of Good Manufacturing Practices. Before administration, cells were thawed, placed on ice, washed in 0·9% NaCl/ X-VIVO 15 medium (1:1 ratio; Lonza Cambrex Bio Science, Verviers, Belgium), and counted in triplicate using the Nucleocouter NC- 200 (ChemoMetec, Denmark). After washing a second time, cells were re-counted to determine the total amount of viable cells and the recovery of the procedure, with a required minimal viability of 70%. MSCs were centrifuged (706 × g at 4 °C for 10 minutes), re-suspended with injection medium (0·9% NaCl/20% human serum albumin; 1:1 ratio) to obtain a concentration of 50 ($\pm$ 10%) × $10^6$ MSCs in 0·6 ($\pm$ 25%) ml, loaded into a 1·0 ml syringe and stored on ice for transport to the clinical ward. To assess the product's stability, three independent MSC formulations were made, stored on ice, and assessed for viability using an NC200 automated cell counter (ChemoMetec A/S, Allerod, Denmark) each hour up to four hours after reconstitution.

MSCs from three different donors remained stable on ice for up to four hours after preparation (viability t=4 relative to t=0; median 96·3% [IQR 94·4-97·9]). Phenotyping using flowcytometry of three different batches of MSCs used in the PASSIoN trial showed that MSC are positive for CD73 (96·5% [96·1-97·1]), CD90 (95·0% [93·9-96·3]), and CD105 (97·6% [97·1-98·4]); and negative for CD3 (lymphocytes; 0·1% [0-0·1]) and CD45 (common leucocyte marker; 1·5% [1·2-1·4]), indicated a high purity of the MSC product. Median recovery after the final formulation steps of preparation was 72·5% [65·4-76·8] and viability remained high after formulation (92·9% [90·3-93·4]). The median time between MSC preparation and administration was 57 [50-61] minutes.

Results

Study population:

The baseline characteristics of the patients and short-term safety of MSC administration were previously described. The infants presented with either clinical or subclinical seizures within 36 hours after birth (table 1), whereafter PAIS diagnosis was confirmed by a neonatal MRI at a median of 3·8 (2·7 - 5·3) days after onset of presenting symptoms. At a median (IQR) age of 6 (1) days after birth, patients received one intranasal dose of $50 \times 10^6$ bone marrow-derived allogeneic MSCs, whereafter no serious adverse events were observed in the first three months after birth.

MRI characteristics:

On the neonatal MRI, the stroke volume ranged between 4 and 24% (median(IQR) 8 (8)%) of the total brain volume). As an additional measure for safety, we examined the extent of tissue loss at three months of age after PAIS using the patient's brain volumes of both neonatal and follow-up MRI scans. The resulting tissue loss ratio (TLR) expresses the amount of tissue lost relative to the volume of the initial volume of the stroke lesion while taking brain growth between both MRI scans into account. A TLR above 100% indicates that more tissue is lost than expected from the initial stroke lesion, and vice versa. The mean±SD TLR of the ten patients was 89±21% and all patients had a TLR below 120% (range 55-114%), which was the predefined per-protocol stopping criterium for safety reasons.

Neurodevelopmental outcome:

At a median age of 24.3 (0.9) months, no serious adverse events or diagnoses of malignancies were reported upon by parents. The neurodevelopmental follow-up measures of the ten PASSIoN children at two years are presented in Table 4. Five (50%) children did not experience an adverse outcome on any of the outcome domains. Two out of ten children (20%) developed cerebral palsy (CP). Both had a GMFCS I classification, with one child having mild bilateral CP affecting the right leg and the left arm, while the other child had unilateral CP with full functionality but reduced movement quality in the hand. Four additional (40%) children exhibited mild tone dysregulation in the extremity contralesional of the hemisphere with the stroke and showed a preference for using the ipsilesional (left) hand, although full functionality was maintained. None of the children had a Bayley motor composite score <85 which would indicate a motor delay, and children started walking at a median (IQR) age of 14 (3) months. Bayley cognitive composite scores were within the normal range for all but one child, who had a score of 82. Two children had a language delay, and one child experienced behavioral problems consisting of sleeping problems. None of the children developed epilepsy or were diagnosed with a visual field defect.

Comparison with registry cohort:

As an exploratory analysis, the characteristics of the neonatal MRI, follow-up MRI, and neurodevelopmental outcome of the participants of the PASSIoN trial were compared to a subset of 39 patients from our registry, born between 1994 and 2022 (Table 3). No statistical differences were observed between the neonatal characteristics of both cohorts (Table 2). Twelve (31%) patients of the registry cohort received Erythropoietin within the first week after birth as part of a safety-feasibility clinical trial. Of the registry cohort, two out of six infants with perinatal asphyxia received therapeutic hypothermia (TH; the other four children were born before TH was available), while one PASSIoN infant had mild perinatal asphyxia without the need for TH.

Both cohorts underwent a neonatal diagnostic MRI scan at a median (IQR) of 4.5 (3) vs. 4 (3) postnatal days (PASSIoN vs. registry cohort, respectively). In the PASSIoN cohort, patients more frequently experienced a middle branch MCA stroke (40%), whereas in the registry cohort, the main MCA stroke was the most common subtype (46%). However, differences in stroke territory distribution between the two groups did not reach statistical significance (p = .06).

On the neonatal MRI, no statistical differences between the two groups concerning the involvement of the perirolandic cortex or the corticospinal tracts were observed, areas both of which are highly predictive of motor outcome. At the follow-up MRI around 3 months of age (postmenstrual age for PASSIoN vs. registry cohort: 53 [2] vs. 53 [3] weeks), patients in the PASSIoN cohort less frequently showed tissue loss in the perirolandic region (50% vs. 92%, p = .007), and asymmetry in the posterior limb of the internal capsule (PLIC; 40% vs. 81%, p = .02) and the cerebral peduncle (10% vs. 61%, p = .01) compared to the registry cohort. No differences were observed in the frequency of asymmetry in the basal ganglia and thalami between the two cohorts.

At neurodevelopmental follow-up around two years of age, less patients from the PASSIoN cohort developed CP than the patients from the registry cohort (20% vs. 49%, p=.16; Table 4). The motor performance score of the PASSIoN cohort was significantly higher than the registry cohort (median Z-score 0.3 (0.8) vs. -0.4 (1.5), p=.003), with 0% and 33% of children having a motor delay, respectively. Additionally, the scores of both cohorts were significantly different from zero in a one-sample t-test (p=.034 and .003, respectively). Children from the PASSIoN cohort started walking at a significantly younger age (p=.006; Table 4). There was no difference regarding hand preference, with 60-74% of the children in both cohorts having a strong preference to use the ipsilesional hand. Less children in the PASSIoN cohort developed a cognitive delay and none developed epilepsy (Table 4). More children of the registry cohort were diagnosed with a visual field defect (0% vs. 41%, p = .02). No differences were observed between the two cohorts in the number of patients with a language delay or a behavioral problem. Since perinatal asphyxia (PA) might affect the degree of neurological injury and neurodevelopmental impairment, we repeated the analyses excluding the six infants with PA from the registry cohort. The results were consistent with the findings described above (data not shown). Other additional neurologic injury detected on MRI (e.g. additional ischemic lesions on neonatal MRI, or global atrophy on follow-up MRI) did not significantly differ between the PASSIoN and the registry cohort on both the neonatal MRI and the follow-up MRI.

Discussion

In this study, the inventors show that IN-MSC therapy after PAIS is safe up to two years of age. The inventors did not observe any long-term negative effects, including serious adverse events, or the occurrence of malignancies in the ten patients that participated in the PASSIoN trial. The ratio of lost tissue after the stroke was 89% on a group level, which indicates that less brain tissue was lost at three months of age than would have been expected based on the initial stroke lesion , and can be seen as an additional confirmation of safety. Furthermore, only two patients (20%) in the PASSIoN cohort developed CP (GMFCS I) at two years of age. All patients had a motor performance within the normal range, including the children with CP. In general, children developed well regarding cognition, epilepsy, language, visual problems, and behavior.

Comparison with registry cohort:

We compared the MRI characteristics and neurodevelopmental outcome of the PASSIoN participants to a registry cohort that met the inclusion and exclusion criteria of the PASSIoN trial.

PASSIoN participants less often showed an asymmetry of the corticospinal tracts on MRI at 3 months of age than the children of the registry cohort, although the initial rates of injury to the corticospinal tracts were similar between groups on the neonatal MRI. At two years of age, patients of the PASSIoN cohort showed a better motor performance, indicated by the motor performance score, the rate of CP, and the age when patients started walking. Inclusion criteria for both PASSIoN and the registry cohort included a MCA stroke involving the cortex, white matter, corticospinal tracts, and basal ganglia. The incidence of CP observed in these types of stroke in literature is 40-66% which is consistent with the rate of CP observed in the registry cohort in our study. However, in PASSIoN infants the rate of asymmetry of the corticospinal tracts on follow-up and the incidence of CP seemed reasonably lower. In the PASSIoN study, only 20% of participants (n=2) developed CP and four additional children had a mild tone difference, which was not classified as having CP. Without being bound to a theory, we speculate that MSC therapy might have stimulated neuroregeneration in the affected motor regions and thereby positively affected motor outcomes and preventing CP. A reduction in CP from 40-66% to 20% could have an important clinical impact on PAIS patients.

Additionally, PASSIoN participants seemed to have a cognitive deficit less often and did not develop epilepsy. In general, non-motor deficits were less often present than motor deficits in both the PASSIoN and registry cohort, although numbers were comparable to literature. Importantly, we reported neurodevelopmental outcomes here up to two years of age, but developmental delays in non-motor domains may manifest later. Assessment of the effect of neuroregenerative therapies on cognition and other non-motor outcomes may therefore require longer follow-up times, although a study by Giraud et al. showed that the age when children with neonatal stroke started walking correlated to cognition scores at seven years of age. In addition, visual field defects were significantly less present in the PASSIoN cohort.

The inventors speculate that MSC treatment efficacy might be dependent on timing after injury but also on the severity of the lesion, as this influences the release of signaling factors, and on the ability of the brain's own neurorepair effects which declines with age.

The results obtained by the inventors in the current invention are the first to demonstrate in newborn humans that intranasal administration of MSCs is effective and safe, in accordance with the invention described herein.

Conclusions

This follow-up study showed that intranasal (IN) MSC therapy for infants with PAIS is safe up to two years of age. In this study, explorative analyses showed results regarding the motor outcome of IN-MSC treated patients with PAIS, which was better compared to a registry cohort and to rates reported in literature. Developing neuroregenerative therapies for infants with PAIS is crucial to improve the quality of life for both the children and their families, since no therapies are currently available.

Having now fully described this invention, it will be appreciated by those skilled in the art that the same can be performed within a wide range of equivalent parameters, concentrations, and conditions without departing from the spirit and scope of the invention and without undue experimentation.

Reference to known method steps, conventional methods steps, known methods or conventional methods is not in any way an admission that any aspect, description, or embodiment of the present invention is disclosed, taught, or suggested in the relevant art.

**Claims**

1.    Mammalian mesenchymal stem cells (MSCs) for use in a method of treatment of a hypoxic-ischemic brain injury (HIBI) event in a human neonate, wherein the method comprises the intranasal administration of an effective amount of mesenchymal stem cells.

2.    The MSCs for use according to the previous claim, wherein the mammalian mesenchymal stem cells are human mesenchymal stem cells, preferably human allogenic mesenchymal stem cells.

3.    The MSCs for use according to any of the previous claims, wherein the human neonate is a neonate of at least 35 weeks gestation.

4.    The MSCs for use according to any of the previous claims, wherein an amount of MSCs from $30 \times 10^6$ to $60 \times 10^6$ cells, preferably from $40 \times 10^6$ to $55 \times 10^6$ cells, and preferably from about $45 \times 10^6$ to $55 \times 10^6$ cells, is administered to the neonate, preferably in a single dose, and preferably by administering nose droplets of a composition comprising the cells over both nostrils and divided equally over the said both nostrils, and preferably with a maximum volume of 0.3 ml per each nostril.

5.    The MSCs for use according to any of the previous claims, wherein the intranasal administration of MSCs is performed within 2-10 days from the HIBI event, preferably within 5 or 6 days from the HIBI event.

6.    The MSCs for use according to any of the previous claims, wherein the HIBI event is a HIBI event caused by a perinatal arterial ischemic stroke (PAIS) and/or a perinatal asphyxia (PA).

7. The MSCs for use according to any of the previous claims, wherein the MSC are selected from the group consisting of bone marrow-derived mesenchymal stem cells (BM-MSC), Umbilical cord mesenchymal stem cells (UC-MSC), and combinations thereof.

8. The MSCs for use according to any of the previous claims, in which the method of treatment is a method of:

- reducing the hypoxic-ischemic injured area after the HIBI event; and/or
- partial or total recovery of tissue in the hypoxic-ischemic injured area after the HIBI event; and/or
- preserving or improving of the cognitive capacity; and/or
- preserving or improving of the motor functionality; and/or
- reducing the incidence or extension of one or more adverse outcome(s) selected from cerebral palsy, epilepsy, language and/or learning disabilities, visual deficits, and behavioral problems.

9. The MSCs for use according to any of the previous claims, wherein the treatment is performed in combination with therapeutic hypothermia, preferably wherein the therapeutic hypothermia is performed at least prior to the intranasal administration of the MSCs.

10. The MSCs for use according to any of the previous claims, wherein the MSCs are obtainable or obtained by:

a) Culturing MSCs, preferably human MSCs, in a cell culture expansion medium, preferably in a cell culture expansion medium comprising the amino acid L-glutamine, heparin and a human platelet lysate, wherein the said culturing in an cell culture expansion medium is carried out for a time to reach a first cell confluence state, to obtain a pool of cells that comprise MSCs that are positive for or that express on their membrane the membrane proteins CD73, CD90, and CD105, and that are negative for or lack expression on their membrane of the membrane proteins CD3, and CD45;

b) optionally passaging the cells of the previous step at least once, preferably two, or three times, preferably in the cell culture expansion medium used in the previous step (a), to reach a second or subsequent cell confluence state;

c) suspending an amount of MSCs obtained in the previous step(s), preferably an amount from $30 \times 10^6$ to $60 \times 10^6$ cells, preferably from $40 \times 10^6$ to $55 \times 10^6$ cells, and preferably from about $45 \times 10^6$ to $55 \times 10^6$ cells, in a medium for intranasal administration, preferably in a liquid (aqueous) medium for intranasal administration, and wherein said (liquid) medium for intranasal administration preferably comprises pharmaceutically acceptable salts and proteins, to obtain a composition for intranasal administration comprising MSCs;

and wherein the obtained composition for intranasal administration comprising MSCs is administered to the human neonate at utmost 4 hours after its obtention.

11. Mammalian mesenchymal stem cells (MSCs) for use in a method of prevention of consequences and/or treatment of a neurodevelopmental disability in a neonate which has suffered a HIBI event, wherein the method comprises the intranasal administration of an effective amount of the MSCs.

12. The MSCs for use according to the previous claim, wherein the neurodevelopmental disability is selected from one or more of cerebral palsy, cognitive dysfunction, epilepsy, visual field deficits, language and/or learning disabilities, and behavioral problems.

13. The MSCs for use in a method of prevention and/or treatment of a neurodevelopmental disability according to any one of claims 11-12,

- wherein the cells are preferably human allogeneic mesenchymal stem cells; and/or
- wherein the human neonate is a neonate of at least 35 weeks gestation; and/or
- wherein the neonate is administered a single dose of MSCs from $30 \times 10^6$ to $60 \times 10^6$ cells, preferably from $40 \times 10^6$ to $55 \times 10^6$ cells, and preferably from about $45 \times 10^6$ to $55 \times 10^6$ cells, and/or
- wherein the intranasal administration is performed within 2-10 days from the HIBI event, preferably within 5 or 6 days from the HIBI event; and/or
- wherein the HIBI event is caused by one or more of a perinatal artery ischemic stroke (PAIS), and a perinatal asphyxia (PA).

14. The MSCs for use in a method of prevention and/or treatment of a neurodevelopmental disability according to any one of claims 11-13, wherein the MSCs are selected from the group consisting of bone marrow-derived mesenchymal stem cells (BM-MSC), umbilical cord mesenchymal stem cells (UC-MSC), and combinations thereof.

15. A method of treating a human neonate subject which has suffered a HIBI event, or a method of preventing and/or treating a neurodevelopmental disability in a neonate which has suffered a HIBI event, the method comprising intranasally administering an effective amount of mammalian MSCs, preferably intranasally administering an effective amount of human MSCs, preferably wherein the MSC are selected from the group consisting of bone marrow-derived mesenchymal stem cells (BM-MSC), Umbilical cord mesenchymal stem cells (UC-MSC), and combinations thereof.

Europäisches
Patentamt

European
Patent Office

Office européen
des brevets

# EUROPEAN SEARCH REPORT

Application Number

EP 24 21 2777

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BAAK LISANNE M ET AL: "Feasibility and safety of intranasally administered mesenchymal stromal cells after perinatal arterial ischaemic stroke in the Netherlands (PASSIoN): a first-in-human, open-label intervention study", THE LANCET NEUROLOGY,, vol. 21, no. 6, 11 May 2022 (2022-05-11), pages 528-536, XP087053474, ISSN: 1474-4422, DOI: 10.1016/S1474-4422(22)00117-X * the whole document * * page 529, left-hand column, paragraph 4 * * page 530, left-hand column, paragraph 4 * * page 530, right-hand column, paragraph 2 * * page 532, right-hand column, paragraph 2-3 * * table 3 and 4 * * page 535, left-hand column, paragraph 2 *  -/-- | 1-8, 10-15 | INV. A61K35/28 C12N5/0775 A61P9/10 A61P25/00 A61P25/28 |
|  |  |  | TECHNICAL FIELDS SEARCHED (IPC) A61K C12N A61P |

The present search report has been drawn up for all claims

| Place of search | Date of completion of the search | Examiner |
|---|---|---|
| Munich | 9 April 2025 | Harrison, Luke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................................

& : member of the same patent family, corresponding document

EPO FORM 1503 03.82 (P04C01)

| | | Europäisches Patentamt<br>European Patent Office<br>Office européen des brevets | **EUROPEAN SEARCH REPORT** | **Application Number**<br>EP 24 21 2777 |
|---|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | & Baak: "Supplement to: Baak LM, Wagenaar N, van der Aa NE, et al. Feasibility and safety of intranasally administered mesenchymal stromal cells after perinatal arterial ischaemic stroke in the Netherlands (PASSIoN): a first-in-human, open-label intervention study. Lancet Neurol 2022; 21: 528-36.", The Lancet Neurology, 1 June 2022 (2022-06-01), XP093263000, Retrieved from the Internet: URL:https://www.thelancet.com/cms/10.1016/ S1474-4422(22)00117-X/attachment/7d791724- 6c59-41fd-8807-478db370320e/mmc1.pdf> * the whole document * * Suppl Information, Research Protocol, Introduction, Final Par * * Suppl Information, Suppl Material, Methods, Para. 1 * * Suppl Information, Suppl Material, Results, Para. 1 * * Suppl Information, Suppl Material, Methods, Para. 2 * * Suppl Information, Research Protocol, Introduction, Final Par. * ----- | | |
| | | | **TECHNICAL FIELDS SEARCHED (IPC)** |
| A | NITKIN CHRISTOPHER R ET AL: "Stem cell therapy for preventing neonatal diseases in the 21st century: Current understanding and challenges", PEDIATRIC RESEARCH, LIPPINCOTT WILLIAMS & WILKINS, NEW YORK, US, vol. 87, no. 2, 14 May 2019 (2019-05-14), pages 265-276, XP036988424, ISSN: 0031-3998, DOI: 10.1038/S41390-019-0425-5 [retrieved on 2019-05-14] * the whole document * ----- | 1-15 | |

The present search report has been drawn up for all claims

| Place of search | Date of completion of the search | Examiner |
|---|---|---|
| Munich | 9 April 2025 | Harrison, Luke |

EPO FORM 1503 03.82 (P04C01)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **COTTEN CM et al.** Feasibility of autologous cord blood cells for infants with hypoxic-ischemic encephalopathy. *J Pediatr.*, 31 December 2013, vol. 164 (5), 973-979
- **TSUJI M et al.** Autologous cord blood cell therapy for neonatal hypoxic-ischaemic encephalopathy: a pilot study for feasibility and safety. *Sci Rep.*, 12 March 2020, vol. 10 (1), 4603
- **AHN SY** ; **CHANG YS** ; **SUNG SI** ; **PARK WS**. Mesenchymal Stem Cells for Severe Intraventricular Hemorrhage in Preterm Infants: Phase I Dose-Escalation Clinical Trial. *Stem Cells Transl Med.*, 21 August 2018, vol. 7 (12), 847-856
- **AMERICAN PSYCHIATRIC ASSOCIATION DIAGNOSTIC**. Statistical Manual of Mental Disorders. 2013
- **VAN BAAR AL** ; **STEENIS LJP** ; **VERHOEVEN M**. Bayley-III-NL; technische handleiding. *Pearson assessment and information*, 2014
- Griffiths Scales of Child Development
- **STROUD, L.** ; **FOXCROFT, C.** ; **GREEN, E.** ; **BLOOMFIELD, S.** ; **CRONJE, J.** ; **HURTER, K. et al.** Griffiths Scales of Child Development. 2016
- Wechsler Preschool and Primary Scale of Intelligence
- Wechsler Intelligence Scale for Children

- **BAAK LM** ; **WAGENAAR N** ; **VAN DER AA NE** ; **GROENENDAAL F** ; **DUDINK J** ; **TATARANNO ML et al.** Feasibility and safety of intranasally administered mesenchymal stromal cells after perinatal arterial ischaemic stroke in the Netherlands (PASSIoN): a first-in-human, open-label intervention study. *Lancet Neurol.*, 2022, vol. 21 (6), 528-36
- **BENDERS MJ et al.** Feasibility and safety of erythropoietin for neuroprotection after perinatal arterial ischemic stroke. *J Pediatr [Internet].*, 04 February 2020, vol. 164 (3), 481-2, http://www.ncbi.nlm.nih.gov/pubmed/24321539
- **KERSBERGEN KJ et al.** Microstructural brain development between 30 and 40 weeks corrected age in a longitudinal cohort of extremely preterm infants. *Neuroimage*, 2014, vol. 103, 214-24, http://dx.doi.org/10.1016/j.neuroimage.2014.09.039
- **ZOETMULDER R et al.** Brain segmentation in patients with perinatal arterial ischemic stroke. *Neuroimage Clin*, 01 January 2023, 38
- **YUSHKEVICH PA et al.** User-guided 3D active contour segmentation of anatomical structures: Significantly improved efficiency and reliability. *Neuroimage*, 01 July 2006, vol. 31 (3), 1116-28
- Griffiths Scales of Child Development. 2009